# EUROPEAN PATENT APPLICATION

(11) **EP 4 502 176 A1**
(43) Date of publication of application: **05.02.2025**
(21) Application number: 23778563.9
(22) Date of filing: 30.03.2023
(51) Int. Cl.: C12Q 1/68, A61P 3/06

(54) **BIOMARKERS AND METHOD FOR DETECTING STATIN INTOLERANCE IN PATIENTS WITH CARDIOVASCULAR RISK**

(30) Priority: 31.03.2022 ES 202230294
(71) Applicant: Universidad de Cádiz, 11003 Cádiz (ES); Servicio Andaluz de Salud, 41071 Sevilla (ES)
(72) Inventor: TORO CEBADA, Rocio, 11003 Cádiz (ES); MANGAS ROJAS, Alipio, 41071 Sevilla (ES); BONET MARTÍNEZ, Fernando, 11003 Cádiz (ES)
(74) Representative: San Martin Alarcia, Esther
(86) International application number: PCT/ES2023/070209
(87) International publication number: WO 2023/187243

(57) **Abstract**

Disclosed are biomarkers that allow statin intolerance to be diagnosed in patients with cardiovascular risk, which is formed by a panel of miRNAs and clinical variables. Also disclosed are a method for detecting statin intolerance, a kit or device, and uses.

## Description

### FIELD OF THE INVENTION

The present invention is within the field of Medicine, and relates to biomarkers and a method for the detection of statin intolerance in patients with cardiovascular risk.

### STATE OF THE ART

Cardiovascular disease is a term used for problems of the heart and blood vessels. The root cause of cardiovascular disease is atherosclerosis, a condition that occurs when cholesterol builds up on the walls of blood vessels.

Cardiovascular disease is the leading cause of death worldwide according to data from the World Health Organization (WHO) referring to 2019, where it is specifically noted that ischemic heart disease is the leading cause of death, being responsible for 16% of all deaths worldwide (https://www.who.int/es/news-room/fact-sheets/detail/the-top-10-causes-of-death).

Atherosclerotic cardiovascular disease (ASCD) is a major component of cardiovascular disease (CVD), which is responsible for more than 4 million deaths annually in Europe (Mach et al. 2020). CVD is also the most common cause of death worldwide. The two most common forms of CVD are coronary heart disease and ischemic stroke, which cause 45% of all annual deaths in Europe: 49% of deaths in women, 40% of deaths in men, and more than 1.4 million premature deaths in people <75 years (Townsend, N. et al. 2016).

In Spain, cardiovascular disease is the most frequent cause of death, ahead of cancer, and hypercholesterolemia accounts for 54% of the potential risk of suffering an acute myocardial infarction (AMI) (Yusuf, S., et al 2004; data from the National Institute of Statistics: https://www.ine.es/ April 2020 and the Spanish Heart Foundation. https://fundaciondelcorazon.*com*/*corazon-facil*/*blog-impulso-vital*/*2208-cuanto-cuesta-enfermo-cardiovascular.html).*

Cardiovascular disease is therefore the main cause of morbidity and mortality worldwide and is especially relevant in the western world where its prevalence compared to other causes of death is much higher, generating an enormous economic cost to the health care system.

Plasma levels of low-density lipids (LDL cholesterol or LDLc) are the main causal factor of these vascular events. In the face of these, statins, or inhibitors of the enzyme hydroxy-methyl-glutaryl-CoA-reductase, are the cornerstone of primary and secondary prevention of atherosclerotic cardiovascular disease worldwide.

Based on evidence from clinical trials, the European Society of Cardiology (ESC)/ European Atherosclerosis Society (EAS) in their 2016 and 2019 "Guidelines on the Management of Dyslipidemias" (https://www.escardio.org/Guidelines/Clinical-Practice-Guidelines/Dyslipidaemias-Management-of) have set LDLc targets or a 50% LDLc concentration reduction in patients at high and very high cardiovascular risk to reduce vascular events.

Although most trials have shown a remarkable, 22% reduction in major vascular events after five years of statin therapy (Cholesterol Treatment Trialists'Collaboration, et al., 2020) almost 80% of treated patients do not reach the recommended LDLc, due to low adherence to therapy.

This low adherence to therapy is due to several causes, including cultural reasons, the price of treatment, side effects and lack of information about its effectiveness (Banach M. et al., 2016).

Although statins are generally well tolerated, the main cause of non-adherence to treatment is precisely the adverse effects derived from statin intolerance, being responsible for non-adherence in patients with cardiovascular risk in up to 15% of cases (Stulc T.et al., 2015; Guyton J.R. et al., 2014; Wai M.Y. et al., 2013).

The European Medicines Agency (EMA) defines in its "Guideline on clinical investigation of medicinal products in the treatment of lipid disorders" (http://www.ema.europa.eu/docs/en_GB/document_library/Scientific_guideline/2014/01 /WC500159540.pdf) statin intolerance as the inability to tolerate at least two or more statins at the lowest approved daily dose, due to the development of side effects that began or increased during statin treatment and ceased when statins were discontinued.

Statin intolerance (SI) presents symptoms related to muscle symptomatology (SAMS), being the most frequent cause of non-adherence and/or discontinuation of statin therapy (Rosenson, R. S., 2014; Guyton J.R. et al., 2014; Wai M.Y. et al., 2013).

Four forms of SAMS have been described: myalgia, myopathy, myositis and rhabdomyolysis. Myalgias or muscle aches, without creatine kinase elevation or significant functional loss, are generally self-reported and constitute 7-29% of cases; myopathies are the most common adverse effect; myositis are muscle inflations with elevation of creatine kinase enzyme concentration; and finally, rhabdomyolysis involves severe muscle damage and is the most severe symptom, estimated at 1-3/100000 patients/year.

The problem in diagnosing statin intolerance is the absence of biomarkers or diagnostic tests that determine which individuals have it, as well as the variability in the design of observational studies, which have not reached any definitive conclusions. The impact of this condition on cardiovascular event rates after discontinuation of statin therapy leads to the need to identify a novel and accessible biomarker that identifies this population (Zhang H,et al., 2017).

To date, the detection of statin intolerance has been based on the presence of abnormal levels of certain markers of liver or muscle function, such as excess creatine kinase. This biomarker is useful only in the case of muscle lysis, which is clearly insufficient, since part of the adverse symptomatology resulting from statin intolerance is not mediated by increased creatine kinase.

Previous studies point to the need to find useful biomarkers for the determination of statin intolerance, as in WO200910683838A1, where the presence or absence of one or more polymorphisms in the SLCOLB1 gene is studied.

Some of these studies point to peripheral blood levels of muscle-specific microRNAs as potential biomarkers for predicting statin-induced skeletal muscle toxicity (Takeshi H. et al, 2020), but without reaching clear conclusions about their role in statin treatments and without providing experimental studies to justify it.

Other studies point to a wide variety of effects of statin application on the expression level of some miRNAs, without a univocal relationship between the presence of statins and their overexpression or underexpression (Zambrano T. et al., 2018), without establishing any direct relationship between these miRNAs levels and the efficacy or toxicity of statins (Ubilla C.G. et al., 2021; Shun S. et al., 2017;) and without knowing the pathways involved (Arrigoni, E. et al., 2017).

In none of these cases are the myopathic effects of statins and circulating levels of miRNAs clearly related, nor are serious candidates among miRNAs proposed as potential biomarkers of statin intolerance.

### DESCRIPTION OF THE INVENTION

We propose a panel of miRNAs and clinical variables as biomarkers for diagnosing patients with statin intolerance.

In addition to avoiding treatment abandonment due to side effects, the identification of this intolerance makes it possible to define which patients with cardiovascular risk are candidates for lipid-lowering drugs, thus avoiding the use of statins in these cases and opting for other alternatives such as anti-PCSK9 inhibitors. It should be borne in mind that all patients under treatment with statins are either in primary prevention (at risk, without an event) or in secondary prevention (have suffered a cardiovascular event such as a vascular accident, vascular disease or coronary heart disease), the latter having a higher cardiovascular risk.

It is therefore a simple diagnosis based on clinical variables taken on a day-to-day basis and miRNAs.

miRNAs are biomarkers that are being used in different fields, such as oncology, because of their characteristics of stability, presence in biological fluids, and because they provide information on epigenetic regulation, metabolic processes, or tissue repair.

MicroRNAs (miRNAs) are small non-coding RNAs about 18-25 nucleotides in length capable of modulating the expression of complementary messenger RNAs by pairing with the untranslated region (3'-UTR) (David P, B., 2004) and have been identified as important regulators of genes involved in various biological processes.

MicroRNAs are single-stranded non-coding RNAs with some secondary structure that negatively regulate (inhibit) gene expression either by inhibition of translation or cleavage of messenger RNA (*mRNA*)*.* Thus, miRNAs are post-transcriptional regulators that bind to complementary sequences of mRNA transcripts, usually resulting in translation repression or target degradation that results in gene silencing.

Under a standard nomenclature system, names are assigned to experimentally confirmed miRNAs as follows: the prefix "mir" is followed (by a hyphen and) a number, whereby the latter can indicate the order of the names.

The uncapitalized "*mir-*" refers to the pre-miRNA, whereas a capitalized "*miR-*" refers to the mature form. MiRNAs with nearly identical sequences, except for one or two nucleotides, are annotated with an additional lowercase letter, e.g. miR-99a. Pre-miRNAs leading to 100% identical mature miRNAs but located at different locations in the genome are indicated with an additional hyphen number suffix. Species of origin can be designated with a three-letter prefix, e.g., hsa-miR-223 is a human (*Homo sapiens*) miRNA. Since, in the context of this paper, all individualized miRNAs are human miRNAs, the prefix *"hsa-"* is sometimes omitted. When two mature miRNAs originate from opposite arms of the same pre-miRNA, they are denoted with a -3p or - 5p suffix, such as miR-142-3p. When relative expression levels are known, an asterisk after the name indicates a miRNA expressed at low levels relative to the miRNA on the opposite arm of a hairpin. Most known miRNA genes are located in intergenic or antisense-oriented regions of neighboring genes and are therefore believed to be transcribed as independent units. Their genes are generally transcribed by RNA polymerase II, and the transcripts are processed, exported from the nucleus and further processed by specific mechanisms, as is well known in the art (see, for example, He et al., 2004).

The miRNA sequences can be accessed at http://www.mirbase.org.

Their properties make them the most studied extracellular RNAs as diagnostic and therapeutic markers in the cardiovascular field (Stepie'n E., 2018; Lu D. et al., 2019).

The use of miRNAs as peripheral biomarkers makes it possible, according to the method of the present invention, to identify the population intolerant to statins and thus personalize the treatment of dyslipidemia (high blood lipid levels) in these patients with cardiovascular risk.

We propose a panel of circulating miRNAs to discriminate statin-intolerant and non-statin-intolerant individuals.

Thus, the term "*miRNA of the invention*" will be used to refer to any miRNA selected from the group consisting of let-7c-5p (SEQ ID No.1), let-7d-5p (SEQ ID No.2), let-7f-5p (SEQ ID No.3), miR-376a-3p (SEQ ID No.4), and miR-376c-3p (SEQ ID No.5).

The set of miRNAs let-7c-5p, let-7d-5p, let-7f-5p, miR-376a-3p and miR-376c-3p present a diagnostic capacity of 0.936 (95% CI: 0.887-0.985; p<0.001).

This, together with clinical variables such as years of dyslipidemia (YDL), the presence of atherosclerotic cardiovascular disease (ASCVD), arterial hypertension, and non-HDL cholesterol levels markedly increase its diagnostic potential.

Therefore, a **first aspect** of the invention relates to the use *in vitro* of the expression levels of let-7c-5p, let-7d-5p, let-7f-5p, miR-376a-3p and/or miR-376c-3p, or any of their combinations, as biomarkers to discriminate statin-intolerant and non-intolerant individuals and thus to predict or prognosticate the occurrence of adverse effects in response to statin treatment in individuals with cardiovascular risk.

In a preferred embodiment of this aspect of the invention, the biomarkers are used simultaneously.

In an even more preferred embodiment of this aspect of the invention, the biomarkers used simultaneously are let-7f-5p, miR-376a-3p and miR-376c-3.

In **another aspect** of the invention, the biomarkers let-7c-5p, let-7d-5p, let-7f-5p, miR-376a-3p and/or miR-376c-3p or any of their combinations constitute expression profiles alone, or together with clinical variables such as years of dyslipidemia, the presence of atherosclerotic cardiovascular disease (ASCVD), arterial hypertension and/or non-HDL-c levels.

In a preferred embodiment of this aspect of the invention, the biomarkers let-7c-5p, let-7d-5p, let-7f-5p, miR-376a-3p and miR-376c-3p are used in combination and the selected clinical variables are years of dyslipidemia and/or non-HDL cholesterol levels.

In an even more preferred embodiment of this aspect of the invention, the biomarkers let-7f-5p, miR-376a-3p and miR-376c-3p are used in combination and the selected clinical variables are years of dyslipidemia and non-HDL cholesterol levels.

### Methods of the invention

Another **aspect** of the invention relates to a method *in vitro* of obtaining data useful for predicting or forecasting the response to statin treatment in individuals with cardiovascular risk, hereinafter first method of the invention, comprising:
a) obtaining an isolated biological sample from the individual,
b) quantifying the expression levels of let-7c-5p, let-7d-5p, let-7f-5p, miR-376a-3p and miR-376c-3p, or any of their combinations.

More preferably the first method of the invention further comprises:
c) comparing the quantities obtained in step (b) with a reference quantity.

Even more preferably, the amount of expression product of all simultaneously will be quantified.

Even more preferably, the amount of let-7f-5p, miR-376a-3p and miR-376c-3p expression product will be quantified simultaneously.

In another preferred embodiment, steps (b) and/or (c) may be fully or partially automated.

Another **aspect** of the invention relates to a method *in vitro* for predicting or forecasting the tolerance/non-tolerance to statins in individuals with cardiovascular risk, hereinafter second method of the invention, comprising steps (a) - (c) according to the first method of the invention, and further comprising:
d) assigning the individual who presents in an analysis, before administering statin treatment, overexpressed expression levels of let-7c-5p, let-7d-5p, let-7f-5p, miR-376a-3p and/or miR-376c-3p, with respect to the mean values of a normal individual, to the group of patients who will suffer adverse effects derived from statin treatment.

A "biological sample" as defined herein is a small part of a subject, representative of the whole. In a preferred embodiment of this aspect of the invention, the biological sample is selected from blood, plasma and serum. In an even more preferred embodiment the biological sample is plasma.

The first method of the invention involves comparing said levels of miRNAs with the levels of said miRNAs of a reference sample or with a median value. In the context of the present invention, "*reference sample*" means the sample that is used to determine the variation of the expression levels of the miRNAs of the present invention. In a preferred embodiment, the reference value is obtained from expression values obtained from a sample with individuals who are not intolerant to statins.

Preferably, reference samples are taken from several individuals who are not intolerant to statins and combined, so that the reference value reflects the average value of such molecules in the population of individuals who are not intolerant to statins. "*Reference value*" means the expression level of a miRNA of the invention in a reference sample.

Detection of the amount of expression product of let-7c-5p, let-7d-5p, let-7f-5p, miR-376a-3p and/or miR-376c-3p, can be performed by any means known in the prior art. The expression levels are going to give a certain profile. The term "expression level", also referred to as "amount of product" or "amount of expression product" refers to biochemical material, specifically miRNA.

The measurement of the amount or concentration of expression product, preferably in a semi-quantitative or quantitative manner, can be carried out either directly or indirectly. Direct measurement refers to the measurement of the amount or concentration of expression product, is directly correlated with the number of RNA molecules. Such a signal (which we may also refer to as an intensity signal) can be obtained, for example, by measuring an intensity value of a chemical or physical property of such products. Indirect measurement includes measurement obtained from a secondary component or a biological measurement system (e.g. measurement of cellular responses, ligands, "label" or enzymatic reaction products).

The term "amount" as used in the description refers to, but is not limited to, the absolute or relative amount of the expression products, as well as any other values or parameters related thereto or which may be derived thereof. Said values or parameters comprise signal intensity values obtained from any of the physical or chemical properties of said expression products obtained by direct measurement. Additionally, said values or parameters include any such values or parameters obtained by indirect measurement, for example, any of the measurement systems described elsewhere herein.

The term "comparison" as used in the description refers to, but is not limited to, the comparison of the amount of the expression products of let-7c-5p, let-7d-5p, let-7f-5p, miR-376a-3p and miR-376c-3p, of the biological sample to be analyzed, also called the biological test sample, with a quantity of the expression products of let-7c-5p, let-7d-5p, let-7f-5p, miR-376a-3p and miR-376c-3p from one or more desirable reference samples. The reference sample can be analyzed, e.g., simultaneously or consecutively, together with the biological test sample. The calculation described in (b) of the method of the present invention can be performed manually or computer-assisted.

In another preferred embodiment of this aspect of the invention, the result can be obtained by any of the following techniques:
(i) a method of generating miRNA profiles, such as a microarray, and/or
(ii) a method comprising PCR (polymerase chain reaction), such as real-time PCR; and/or
(iii) Northern transfer, and/or
(iv) immunoassay.

In the invention, the method for determining the result, i.e., the level of miRNA expression, need not be particularly limited, and may be selected by a miRNA profiling method, such as a microarray, and/or a method comprising PCR (polymerase chain reaction), such as PCR time; and/or Northern Blot.

Real-time quantitative PCR (polymerase chain reaction) (generally abbreviated as RQ-PCR, RT-qPCR, rt-PCR or qPCR) is a sensitive and reproducible miRNA expression quantification technique that can be used particularly for profiling miRNA expression in cells and tissues. Any method can be used to evaluate RT-PCR results, and the ΔCt method and the ΔΔCt method may be preferred. The ΔΔCt method is described in detail by Livak et al. (Methods 2001, 25: 402-408). (Ct = Cycle threshold values). In implementing the present invention, the ΔΔCt method described by Livak et al. (Methods 2001, 25: 402-408) will be used preferably. The ΔΔCt-method will include a 'control sample' and a 'subject sample'. The 'subject sample' is a sample of the subject to be analyzed. For each sample, a target miRNA (here: miRNA of interest) and an endogenous control miRNA (as described below) are included for PCR amplification from aliquots (typically serially). Typically, several replicates for each diluted concentration are used to derive amplification efficiency. PCR amplification efficiency can be defined as percentage amplification (from 0 to 1). During the qPCR reaction, a software typically measures for each sample the cycle number at which the fluorescence (PCR amplification indicator) crosses an arbitrary line, the threshold. This crossing point is the Ct value.

A microarray is an array on a solid substrate (usually a glass slide or a silicon thin film cell) that analyzes large amounts of biological material, in the present case a large amount of different miRNAs or, preferably, their reverse DNA transcripts, which are detectable by specific probes immobilized on the solid substrate.

A Northern transfer involves the use of electrophoresis to separate RNA samples by size and subsequent detection with a hybridization probe complementary to (part of) the target sequence of the RNA of interest.

The term "immunoassay" as used in the present description refers to any analytical technique that is based on the conjugation reaction of an antibody to an antigen. Examples of immunoassays known in the prior art are, for example, but not limited to: immunoblot, enzyme-linked immunosorbent assay (ELISA), linear immunoassay (LIA), radioimmunoassay (RIA), immunofluorescence, x-map or protein chips.

In another preferred embodiment, the immunoassay is an *Enzyme-Linked ImmunoSorbent Assay* (ELISA). ELISA is based on the premise that an immunoreagent (antigen or antibody) can be immobilized on a solid support, then bringing that system into contact with a fluid phase containing the complementary reagent that can bind to a marker compound. There are different types of ELISA: direct ELISA, indirect ELISA or sandwich ELISA. The term "marker compound" as used in the present description refers to a compound capable of giving rise to a chromogenic, fluorogenic, radioactive and/or chemiluminescent signal that allows detection and quantification of the amount of antibodies against let-7c-5p, let-7d-5p, let-7f-5p, miR-376a-3p and miR-376c-3p. The marker compound is selected from the list comprising radioisotopes, enzymes, fluorophores or any molecule susceptible to be conjugated to another molecule or directly detected and/or quantified. This marker compound can bind to the antibody directly, or through another compound. Examples of marker compounds that bind directly include, but are not limited to, enzymes such as alkaline phosphatase or peroxidase, radioactive isotopes such as 32P or 35S, fluorochromes such as fluorescein, or metal particles, for direct detection by colorimetry, autoradiography, fluorimetry, or metallography, respectively.

The method of the present invention can be applied with samples of individuals of either sex, i.e., males or females, and at any age.

In the method of the present invention, the expression of miRNA can be normalized, preferably relative to the expression of another RNA molecule. There are well known normalization methods in the prior art.

The invention provides a method for assigning a human subject into one of two groups: group 1, comprising subjects identifiable by the method of the invention and group 2, representing the remaining subjects.

It is possible to provide personalized therapy to an individual depending on whether the individual is assigned to group 1 or group 2. Group 1 comprises subjects identifiable by the method of the invention as individuals suffering from statin intolerance and group 2 represents the remaining subjects.

A "reference sample," as used herein, means a sample obtained from a group of healthy subjects that does not have a particular disease state or phenotype.

Reference levels can be determined by measuring expression levels, and those reference levels can be adjusted to specific populations (e.g., a reference level can be age-related, so that comparisons can be made between expression levels in samples from subjects of a certain age and reference levels for a particular disease, phenotype, or lack thereof in a certain age group). In a preferred embodiment, the reference sample is obtained from various subjects in general, or from subjects not intolerant to statins.

The person skilled in the art will appreciate that the type of reference sample may vary depending on the specific method to be performed.

The expression profile in the preferred reference sample can be generated from a population of two or more persons. The population, for example, may contain 3, 4, 5, 10, 15, 15, 20, 30, 40, 50 or more persons.

Once the expression levels relative to the reference values have been determined, it is necessary to identify whether there are alterations in expression (increased or decreased expression). Expression (and gene expression product levels) is considered increased in a test subject sample when the levels of increase relative to the reference sample are at least 5%, at least 10%, at least 15%, at least 20%, at least 25%, at least 30%, at least 35%, at least 40%, at least 45%, at least 50%, at least 55%, at least 60%, at least at least 65%, at least 70%, at least 75%, at least 80%, at least 85%, at least 90%, at least 95%, at least 100%, at least 110%, at least 120%, at least 130%, at least 140%, at least 150%, or more. Similarly, the expression is considered decreased when its levels decrease with respect to the reference sample by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100% (i.e. absent).

### Kit or composition of the invention and uses

Another **aspect of the invention** relates to a kit or device, hereinafter kit or device of the invention, comprising elements necessary to quantify the expression level of let-7c-5p, let-7d-5p, let-7f-5p, miR-376a-3p and/or miR-376c-3p, or any combination thereof.

Preferably it comprises the elements necessary to simultaneously quantify let-7c-5p, let-7d-5p, let-7f-5p, miR-376a- 3p and miR-376c-3p.

Even more preferably it comprises the elements necessary to simultaneously quantify let-7f-5p, miR-376a-3p and miR-376c-3p.

In a preferred embodiment the kit or device of the invention comprises:
(a) means for detecting in a biological sample obtained from the subject the expression levels of the biomarkers of the invention,
(b) means to compare the level of expression of the biomarkers determined in (a) with a reference sample,
(c) instructions for a medical professional to administer statin treatment only to those subjects who can be classified in the non-statin intolerant group.

In a preferred embodiment, the kit or device comprises at least one or more oligonucleotides capable of hybridizing with miRNAs let-7c-5p, let-7d-5p, let-7f-5p, miR-376a-3p and/or miR-376c-3p, or any combination thereof.

It is preferred that said oligonucleotide(s) be capable of doing so under astringency conditions. In a preferred embodiment, one or more of said one or more oligonucleotides (preferably DNA) are further defined by the following probes or primers: hsa-miRXX-miRCURY LNA miRNA measured with syber, specific LNA ^{™} PCR primers.

Astringency is a term used in hybridization experiments. Astringency reflects the degree of complementarity between the oligonucleotide and the nucleic acid (which in this case is the miRNA to be detected); the higher the astringency, the higher the percentage of homology between the probe and the nucleic acid bound to the filter. It is well known to the expert in the art that temperature and salt concentrations have a direct effect on the results obtained. It is recognized that hybridization results are related to the number of degrees below the Tm (melting temperature) of the DNA at which the experiment is performed. Often, stringent conditions are defined as a wash with 0.1X SSC (sodium saline-sodium citrate (SSC) buffer at 65 °C. (SSC is usually provided as a 20X stock solution, consisting of 3 M sodium chloride and 300 mM trisodium citrate (adjusted to pH 7.0 with HCI)).

In particular embodiments, the kit is selected from (a) a kit suitable for PCR, (b) a kit suitable for Northern Blot, (c) a kit suitable for microarray analysis and (d) an immunoassay. Two or more of these realizations may also be combined, so that the kit may comprise, for example, both (a) and (c).

In the case of (a) a kit suitable for PCR, this PCR is typically a real-time quantitative PCR (RQ-PCR), a sensitive and reproducible gene expression quantification technique. In this case, it is desired that the kit additionally comprise a polyT oligonucleotide primer in addition to the oligonucleotide(s) in the kit. The polyT oligonucleotide primer polyT can be used together with the oligonucleotide(s) of the invention for priming PCR, after polyadenylation of the isolated miRNAs by methods known to experts, such as the use of poly(A) polymerase and ATP. These reagents may optionally be included in the kit.

A Northern transfer involves the use of electrophoresis to separate RNA samples by size and subsequent detection with an oligonucleotide(s) (hybridization probe) complementary to (part of) the RNA target sequence of interest.

It is also possible for the oligonucleotide(s) to be immobilized on spots on a (preferably solid) surface. In one embodiment, the kit comprises a microarray. An RNA microarray is an array on a solid substrate (usually a glass slide or a silicon thin film cell) that analyzes large amounts of different RNAs (in this case miRNA), which can be detected by specific probes immobilized on spots on the solid substrate. Each spot contains a specific nucleic acid sequence, typically a DNA sequence, known as probes (or reporters). While the number of spots is not limited, there is a preferred embodiment in which the microarray is adapted to the methods of the invention. In one embodiment, such a customized microarray comprises fifty spots or less, such as thirty spots or less, including twenty spots or less.

The kit or device of the invention may be used and the use is not particularly limited, although use in the method of the invention in any of its embodiments is preferred.

In another preferred embodiment of this aspect of the invention, the oligonucleotides, primers, probes or antibodies are modified or labeled, for example, but not limited to, by radioactive or immunological labeling. Thus, preferably, the oligonucleotides have modifications in one of their nucleotides, such as, but not limited to, nucleotides having one of their atoms with a radioactive isotope, typically ³²P or tritium, immunologically labeled nucleotides, such as with a digoxigenin molecule, and/or immobilized on a membrane. Several possibilities are known in the prior art.

The kit or device of the invention may comprise controls, program instructions and information necessary to carry out any of the methods of the invention.

Another **aspect of the invention** relates to the use of any of the kits or devices of the present invention, in obtaining data useful in predicting or predicting intolerance to statin treatment in an individual, preferably where the individual presents a with high cardiovascular risk.

### Automation of the method of the invention by implementing it in a computer program.

Another **aspect of the invention** relates to a computer program comprising instructions for performing the procedure according to any of the methods of the invention.

In particular, the invention encompasses computer programs arranged on or within a carrier. The carrier may be any entity or device capable of supporting the program.

Where the program is embodied in a signal which can be carried directly by a cable or other device or medium, the carrier may consist of such a cable or other device or medium. As a variant, the carrier could be an integrated circuit in which the program is embedded and which has been adapted to execute, or to be used in the execution of, the corresponding processes.

For example, the programs could be embodied in a storage medium, such as a ROM memory, a CD ROM or semiconductor ROM memory, a USB memory, or a magnetic recording medium, e.g., a floppy disk or a hard disk. Alternatively, the programs could be supported on a transmittable carrier signal; for example, this could be an electrical or optical signal that could be carried via electrical or optical cable, by radio, or by any other means.

The invention also extends to computer programs adapted so that any processing means can implement the methods of the invention. Such programs may be in the form of source code, object code, an intermediate source of code and object code, for example, as in partially compiled form, or in any other form suitable for use in the implementation of the processes according to the invention. Computer programs also comprise cloud applications based on said procedure.

Other aspects of the invention relate to the readable storage medium and to the transmittable signal comprising program instructions necessary for execution of the method of invention by a computer.

Thus, another aspect of the invention relates to a computer-readable storage medium comprising program instructions capable of causing a computer to carry out the steps of any of the methods of the invention.

Throughout the description and the claims the word "comprises" and variants thereof are not intended to exclude other technical features, additives, components or steps. To those skilled in the art, other objects, advantages and features of the invention will be apparent in part from the description and in part from the practice of the invention. The following examples and drawings are provided by way of illustration, and are not intended to be limiting of the present invention.

### DESCRIPTION OF THE FIGURES

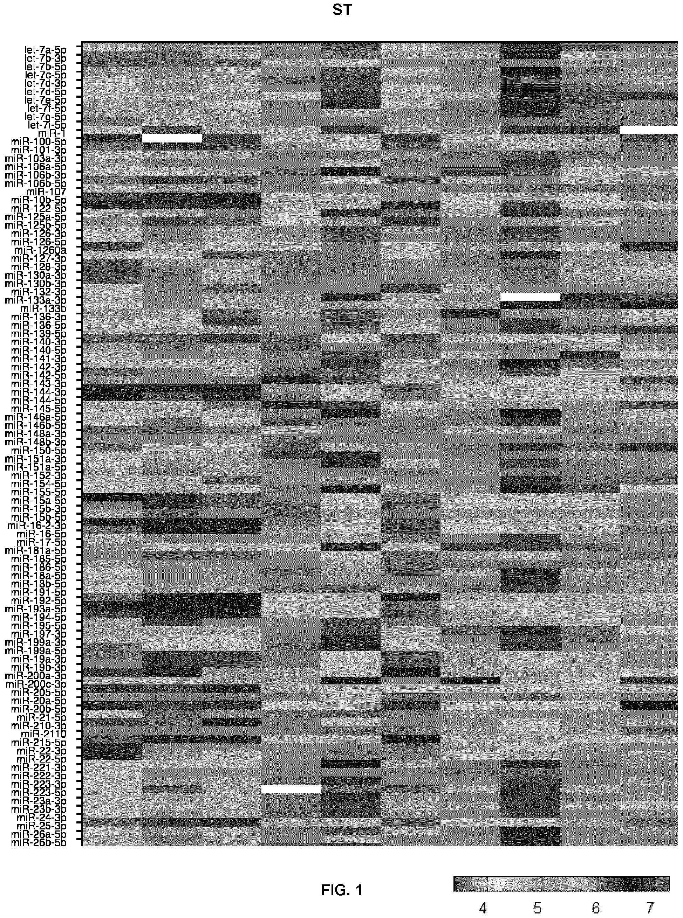

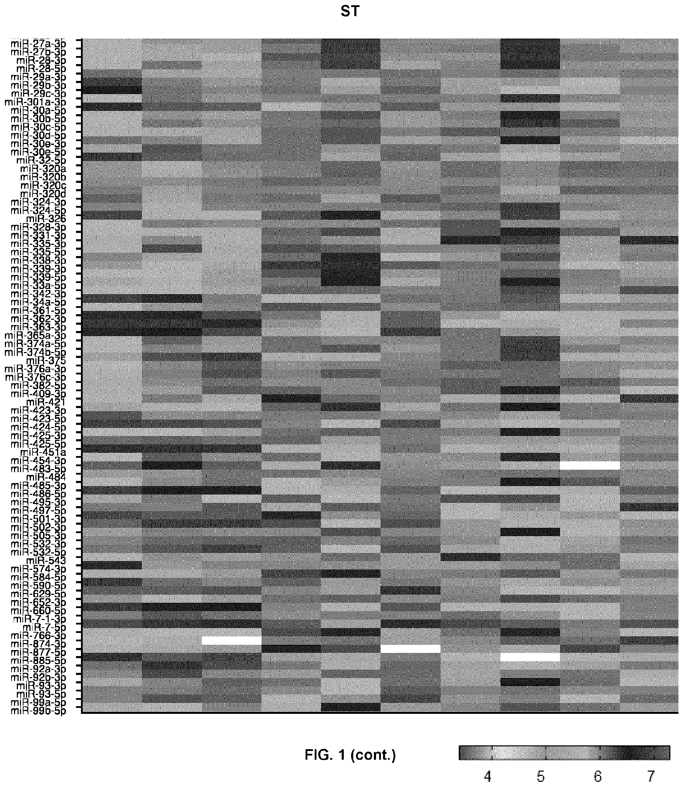

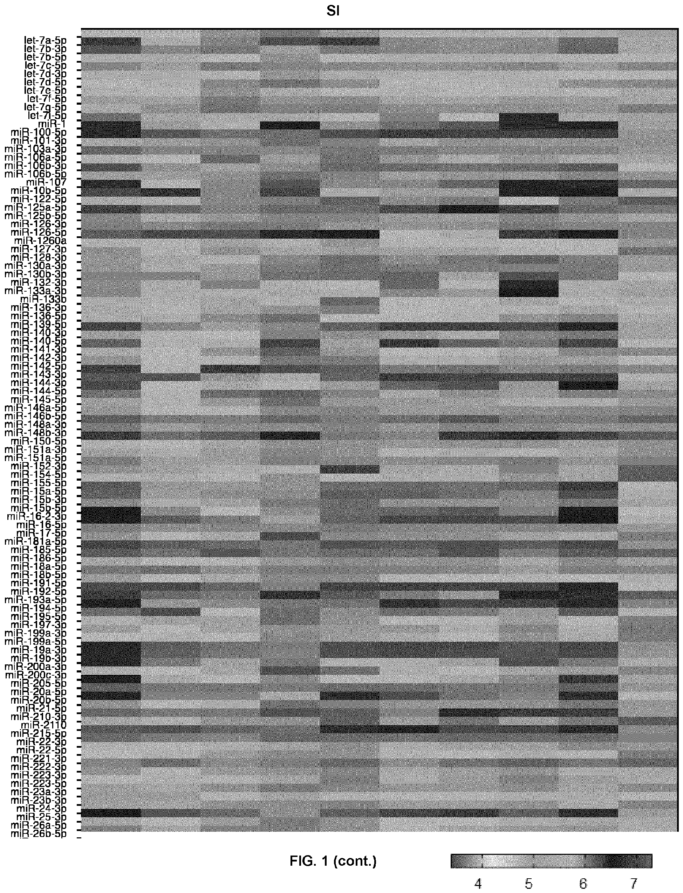

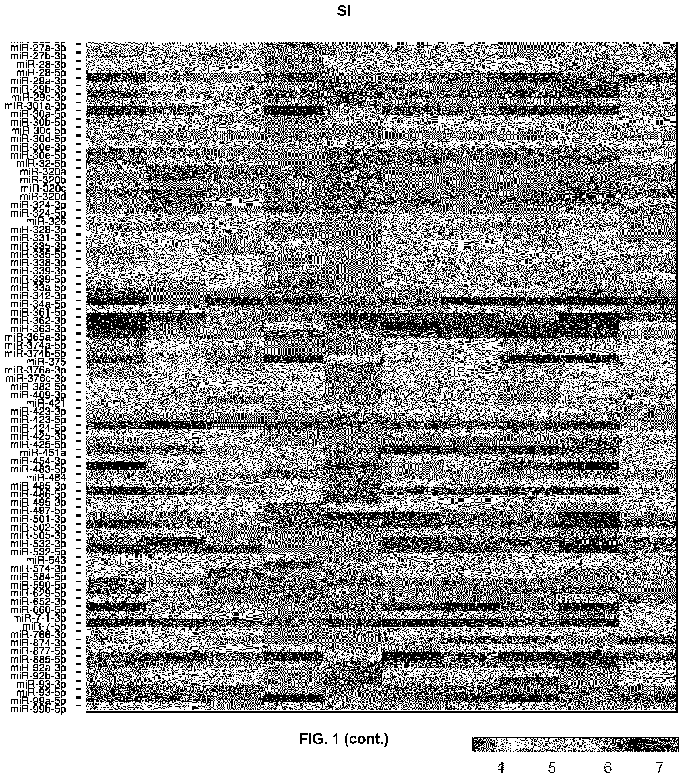

**Fig. 1****.** Plasma microRNA profiles of statin-tolerant (ST) (n = 10) and statin-intolerant (SI) (n = 10) patients. 6-color heat map based on raw miRNA expression values (red and yellow represent low expression and blue and purple represent high expression). The miRNA expression levels were normalized to miR-148a-3p and hsa-let-7b-5p.

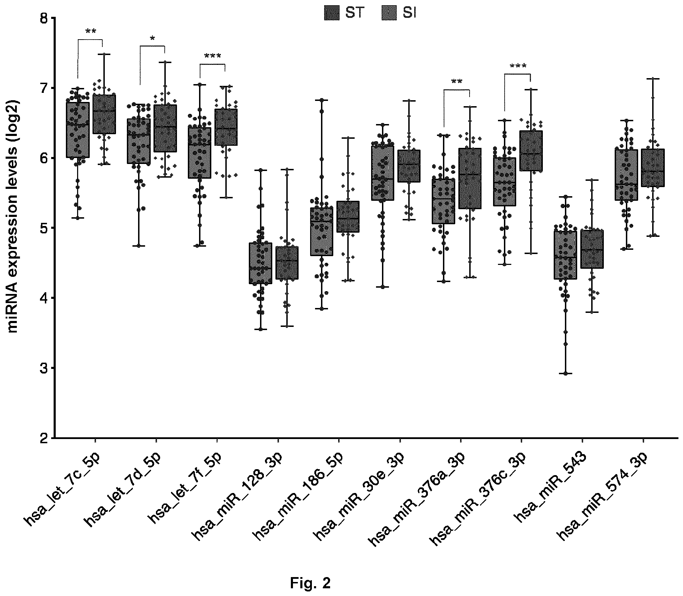

**Fig. 2****.** Box plots of miRNA expression levels normalized to miR-148a-3p in ST statin-tolerant (n = 45) and SI statin-intolerant (n = 39) subjects. Analysis was performed by qPCR. Data are presented in log2. Data represent mean ± SEM. *p < 0.05, **p < 0.01, ***p < 0.005. Each paper represents one patient. Error bars represent standard deviation (SD).

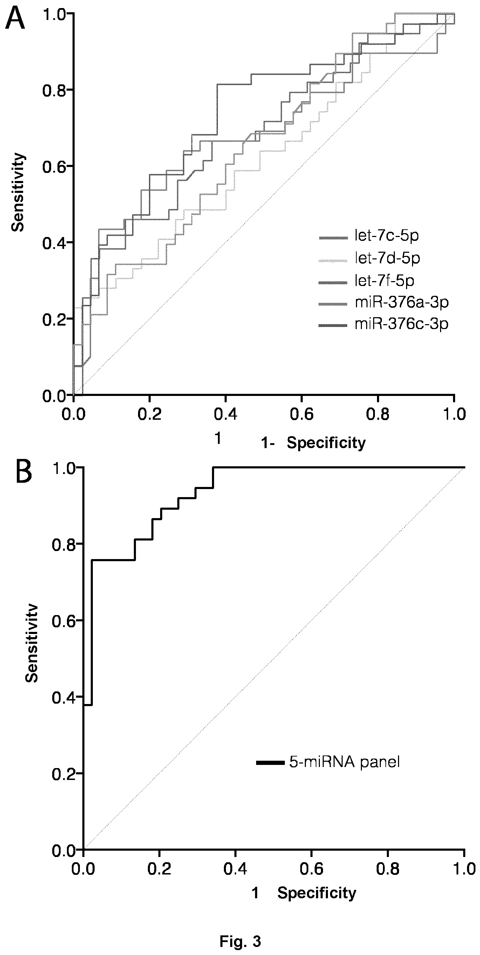

**Fig. 3****.** ROC curves to evaluate the predictive performance of differentially expressed miRNAs. (A) ROC curves for let-7c-5p, let-7d-5p, let-7f-5p, miR-376a-3p and miR-376c-3p. (B) The ROC curve of the panel of 5 miRNAs, i.e. the combination value of let-7c-5p, let-7d-5p, let-7f-5p, miR-376a-3p and miR-376c-3p.

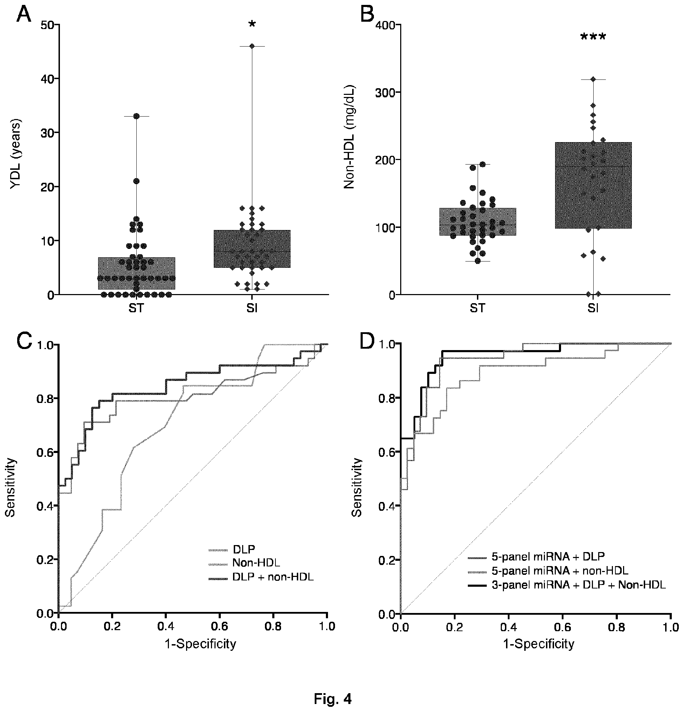

**Fig. 4****.** ROC curves to evaluate the predictive performance of clinical parameters in combination with differentially expressed miRNA. (A) Box plot of years of dyslipidemia (YDL) in statin-tolerant (ST) (n = 45) and statin-intolerant (SI) (n = 39) subjects. (B) Box plot of non-HDL-c levels in statin-tolerant (ST) (n = 45) and statin-intolerant (SI) (n = 39) subjects. (C) ROC curves for each clinical parameter, YDL and non-HDL-c, and for the combination of YDL plus non-HDL-c. (D) ROC curve of the 3-miRNA (let-7f-5p, miR-376a-3p and miR-376c-3p), YDL and non-HDL-c panel combination value.

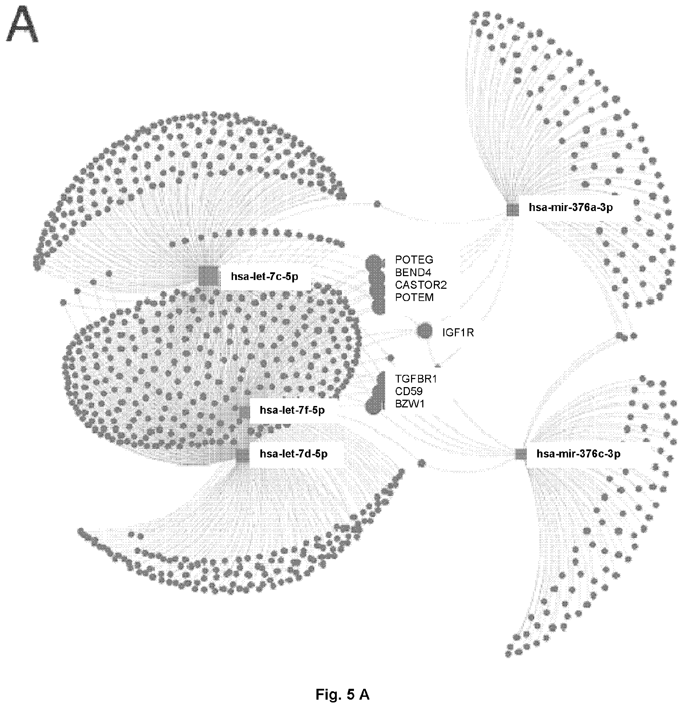

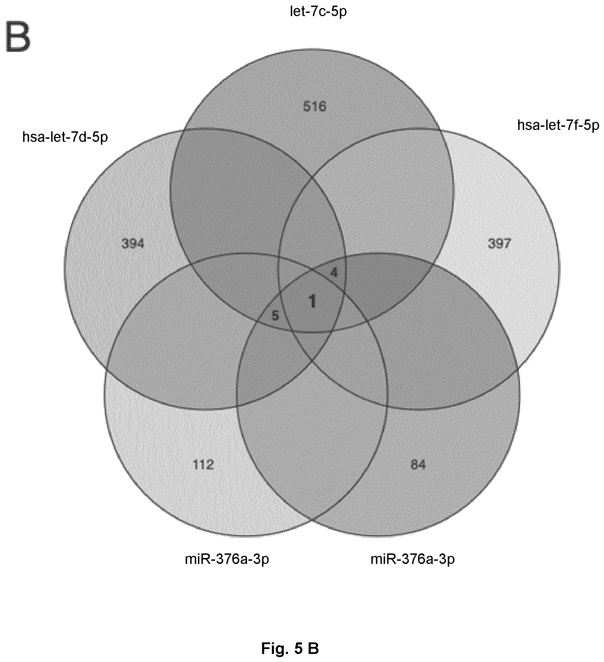

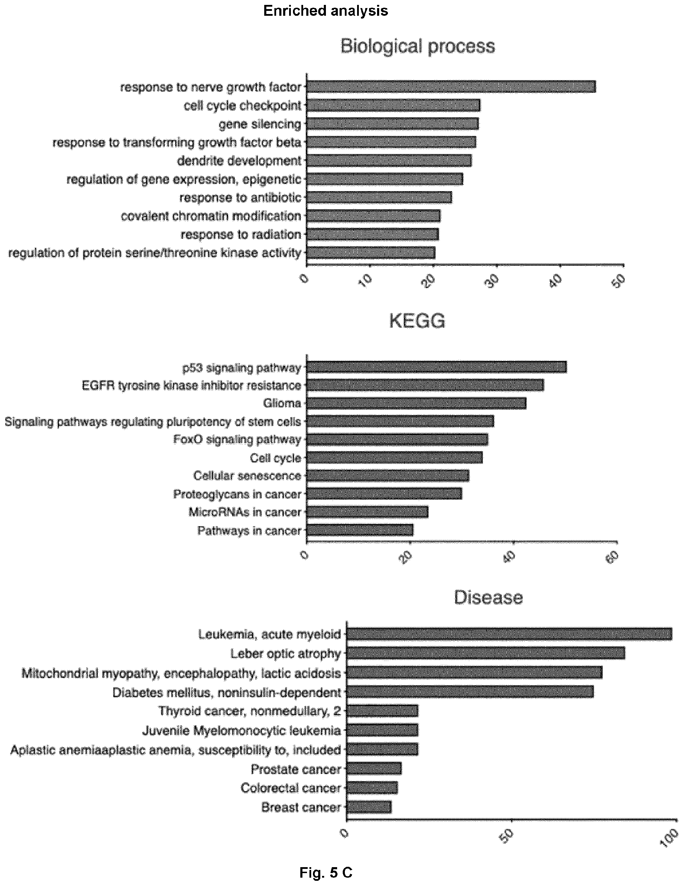

**Fig. 5****.** KEGG (Kyoto Encyclopedia of Genes and Genomes) and GO (gene ontology) analysis of differentially expressed miRNAs. (A) miRNA gene network for let-7c-5p, let-7d-5p, let-7f-5p, miR-376a-3p and miR-376c-3p. (B) Venn diagram showing gene target overlap of let-7c-5p, let-7d-5p, let-7f-5p, miR-376a-3p and miR-376c-3p. (C) GO and KEGG functional enrichment analysis of let-7c-5p, let-7d-5p, let-7f-5p, miR-376a-3p and miR-376c-3p.

### EXAMPLES OF THE INVENTION

### Population study and design

This was a prospective multicenter study. Patients were consecutively recruited from three centers (Hospital Universitario Puerta del Mar, Cádiz; Hospital Universitario Virgen del Rocío, Seville; and Hospital Universitario Reina Sofía de Córdoba, Spain).

The inclusion criteria were: patients over 18 years of age with high and very high cardiovascular risk in treatment with statins who had been referred to the Lipid Unit of these centers.

A total of 82 consecutive subjects divided into two different groups were included: i) statin intolerant (SI) patients (n=37) and, ii) statin tolerant (ST) patients (n=45). Statin intolerance (SI) was defined according to the criteria of the European Medicines Agency (*Guideline on clinical investigation of medicinal products in the treatment of lipid disorders;* http://www.ema.europa.eu/docs/en_GB/document_library/Scientific_guideline/2014/01/ WC500159540.pdf). All patients were classified according to the cardiovascular risk grade of the 2019 ESC/EAS guideline for the management of dyslipidemias (https://www.escardio.org/Guidelines/Clinical-Practice-Guidelines/Dyslipidaemias-Management-of).

Patients with clinically documented or unequivocal imaging evidence of atherosclerotic cardiovascular disease (ASCVD), patients with diabetes mellitus (DM) with white organ damage or at least three major risk factors, or early-onset type 1 diabetes mellitus lasting more than 20 years, severe chronic kidney disease (CKD) (estimated glomerular filtration rate [eGFR < 30 ml7min/1.73 m2] or a calculated SCORE equal to/superior to 10% for 10 years of fatal CVD risk and familial hypercholesterolemia (FH) with an ASCVD or with another major risk factor, were considered as at very high cardiovascular risk. Patients with a single markedly elevated risk factor were defined as high cardiovascular risk, in particular those with total cholesterol above 310 mg/dl, LDLc above 190 mg/dl, or blood pressure (BP) equal to/above 180/110 mg/dl, FH without another major risk factor, patients with DM without target organ damage, DM of more than 10 years of evolution or another additional risk factor, moderate CKD (eGFR: 30-59mL7min/1.73 m2) or SCORE calculated ≥5 % and <10% f or 10 years-risk of fatal CVD.

Exclusion criteria were patients over 80 years of age or marked frailty, intense physical activity, high alcohol consumption, patients with chronic inflammatory, autoimmune or tumor disease or other pharmacological therapy that interfered with the metabolic pathways of the different statins. Subjects with previous muscle symptoms, myopathic disease, elevated creatine kinase levels or hypothyroidism were also excluded from the study.

Detailed anthropometric, clinical and pharmacological information was obtained for each subject, including cardiovascular risk factors and previous vascular events. All patients underwent blood analysis including thyroid, hepatic, renal and lipid profiles, as well as creatine kinase. The ethics committee of our institution (Cadiz Research Ethics Committee) approved the study protocol. The study was conducted in full compliance with the Declaration of Helsinki II. All participants gave written informed consent.

### Blood collection

Ten milliliters of peripheral blood were collected in K2-ethylenediaminetetraacetic acid (BD) tubes after 10h of overnight fasting and centrifuged immediately (1500 xg, 15 min, 4°C). Blood was processed within 4h after isolation. The upper layer containing plasma was divided into aliquots and stored at -80°C until further analysis.

### RNA isolation

The miRNeasy Serum/Plasma kit (Qiagen) was used to extract total RNA according to the manufacturer's instructions. A total of 200 µL of plasma aliquots were used to obtain a sufficient final volume. As internal amplification controls, synthetic miRNA from C. Elegans (cel-miR-39-3p) (1.6 × 10⁸ copies/µL) (Qiagen) and UniSp6 (Qiagen) were added prior to extraction.

### miRNA real time reverse transcriptase polymerase chain reaction

To assess miRNA expression levels and SI and ST group profiles, analysis of 179 microRNAs from miRNA species known to be present in human serum was performed using the miRCURY LNA RT kit (Qiagen) for reverse transcription, the miRCURY LNA SYBR Green PCR kit (Qiagen) for quantitative PCR amplification (qPCR) and Focus human microRNA PCR panels in serum/plasma (Exiqon) according to the manufacturer's instructions. Raw cycle threshold (Cq) values were calibrated between plates using UniSp3. Cqs above 35 cycles were censored at the minimum observed level for each miRNA. As the cleanup miRNA for data normalization, we selected the most stably expressed miRNA pair (miR-148a-3p and let-7b-5p) as determined by the Normfinder algorithm (Andersen et al., 2004). Accordingly, each individual miRNA was normalized as ΔCq = mean(CqmiR-148a-3p,let-7b-5p) - CqmiRNA.

The miRNA levels were logarithmically transformed before being used in statistical analyses.

RNA was reverse transcribed using the miRCURY LNA RT kit (Qiagen). The qRT-PCR was performed with the miRCURY LNA SYBR Green PCR kit (Qiagen) as previously described (Belmonte et al., 2020) and amplification curves were evaluated with CFX Manager^{™} software (BioRad). Amplification specificity was corroborated by melting curve analysis.

### miRNA gene network analysis

The obtained miRNAs were analyzed using the miRNet database (https://www.mirnet.ca) to predict target genes. Database analysis was performed to identify biological function using Gene Ontology (GO) enrichment analysis (http://geneontology.org/) (Huaiyu M et al, 2019) and Kyoto Encyclopedia of Genes and Genomes (KEGG) (http://www.genome .jp/kegg/). The search tool for retrieval of interacting gene database (STRING) (http://www.string-db.org/) was used to analyze protein-protein interaction (PPI) networks (Szklarczyk, D. et al., 2019).

### KEGG and GO term enrichment analysis

The miRNet online software (https://www.mirnet.ca) was used to predict target genes and construct the miRNA-mRNA regulatory network. The WebGesalt computational tool (Web-based GEne SeT AnaLysis Toolkit) for KEGG pathway and GO term analysis. GO term analysis was used to determine the involvement of the 5 differentially expressed miRNAs in biological process and disease.

### Statistical analysis

Continuous variables are shown as mean ± standard deviation (SD). Categorical variables are expressed as frequency and percentage of patients (%).

Between-group comparisons of miRNA levels were performed by nonparametric Mann-Whitney and Kruskal-Wallis rank tests for continuous variables. Analysis of differences between groups was performed by analysis of variance.

ROC curves characterizing the diagnostic performance of candidate miRNAs and logistic regression models were plotted to determine the area under the curve (AUC) and the specificity and sensitivity of optimal cutoff points. ROC curves were generated by plotting sensitivity versus specificity 1. Data were presented as AUC and 95% confidence intervals. Changes in the p values of their variables were evaluated using the Wald test and a likelihood ratio. The R statistical software package (www.r- project. org) was used for all analyses (Team RC. R: A Language and Environment for Statistical Computing. https://www.r- project.org).

### RESULTS

### Clinical parameters between SI and ST patients

The anthropometric and clinical characteristics of the statin-intolerant (SI) and statin-tolerant (ST) cohorts are shown in Table 1. A total of 84 patients were enrolled in this study, 39 SI patients and 45 ST patients in the control group. The subjects showed no significant differences in age. Between groups, the male population was larger than the female population, however, female SI cases (61.5%) were significantly more frequent (38.5%) (p = 0.01); in contrast, a lower percentage of females (31%) was observed in the ST group. Regarding previous cardiovascular data, the presence of atherosclerotic cardiovascular disease (ASCVD), years of dyslipidemia (YDL) (p = 0.003) and arterial hypertension (p = 0.006) were significantly different between groups. On the other hand, non-HDL-c plasma concentration (p < 0.001) showed significant differences between the SI and ST groups. Finally, regarding the assessment of medication intake, the use of angiotensin receptor agonist (ARB) (p = 0.004), diuretics (p = 0.003), beta blockers (p = <0.001), alpha blockers (p = 0.03) and aspirin (p = <0.001) there were significant differences between the SI and ST cohorts.

**Table 1. Baseline demographic and clinical characteristics of patients with ST and SI.**

| Data presented as mean ± SD for continuous variables and as percentage for categorical variables. The difference between patients with ST and SI was evaluated with unpaired Student's t test, Pearson Chi-Squareb and Wilcoxon testc. | | | |
|---|---|---|---|
| NS, not significant; ASCVD, atherosclerotic cardiovascular disease; ARA II, angiotensin II receptor antagonist; MDRD-4, glomerular filtration rates; CPK, creatine phosphokinase; ACE, angiotensin-converting enzyme; ARB, angiotensin II receptor blockers; OAD, oral antidiabetic drugs; CCB, calcium channel blockers; ASA, aspirin. | | | |
| **Variables** | **ST** | **SI** | **p value** |
| ***n*** | 45 | 39 | |

| **Anthropometric** | | | |
|---|---|---|---|
| Age (years)^{a} | 66.6 ± 11.5 | 63.6 ± 10.9 | NS |
| Sex (male, %)^{b} | 31 | 61.5 | 0.01 |
| Dyslipidemia (years)^{c} | 5.6 ± 6.3 | 9.1 ± 7.5 | 0.003 |
| High blood pressure (%)^{b} | 73 | 41 | 0.006 |

| **Cardiovascular risk** | | | |
|---|---|---|---|
| Diabetes Mellitus (%)^{b} | 38 | 20.5 | NS |
| ASCVD (%)^{b} | 71 | 13 | <0.001 |
| Chronic kidney disease (%)^{b} | 18 | 12,8 | NS |

| **Analytical profile** | | | |
|---|---|---|---|
| Basal blood glucose^{a} | 118.5 ± 36.2 | 110 ± 44.6 | NS |
| Non-HDLc (mg/dL)^{a} | 104.9 ± 32.7 | 169.9± 63.5 | <0.001 |
| Triglycerides (mg/dL)^{a} | 151.7 ± 97.4 | 164 ± 93 | NS |
| MDRD-4 (mL/min)^{a} | 76.5 ± 25.6 | 82 ± 31.6 | NS |
| Transaminase GOT (U/L)^{a} | 23.1 ± 13 | 23.5 ± 8 | NS |
| Transaminase GPT (U/L)^{a} | 27.3 ± 23 | 24.6 ± 18.9 | NS |
| CPK (U/L)^{a} | 82.8 ± 40.4 | 165.9 ± 14.7 | NS |

| **Treatment (%)^{b}** | | | |
|---|---|---|---|
| Ezetimibe | 42 | 38 | NS |
| Fibrates | 18 | 7.7 | NS |
| ACE inhibitors | 11 | 2.5 | NS |
| ARBs | 67 | 33.3 | 0.004 |
| OADs | 33 | 18 | NS |
| Insulin use | 11 | 10.5 | NS |
| Diuretic | 51 | 18 | 0.003 |
| CCBs | 31 | 18 | NS |
| Beta-blockers | 62 | 15.4 | <0.001 |
| Alpha-blockers | 22 | 5 | 0.03 |
| ASA | 64 | 18 | <0.001 |
| Use of Acenocoumarol | 7 | 7.6 | NS |

### Plasma microRNA profiling in patients with SI

To assess whether plasma miRNAs were differentially expressed between groups, we first analyzed the profile of 179 circulating miRNAs commonly found in human plasma in age-matched patients with 10 SI and 10 ST (Figure 1). The criteria established for selection of miRNA candidates were high expression levels (median Cq < 32 and detected in at least 80% of all samples) and statistical significance (p<0.05). A total of 10 miRNAs, let-7c-5p, let-7d-5p, let-7f-5p, miR-128-3p, miR-186-5p, miR-30e-3p, miR-376a-3p, miR-376c-3p, miR-543 and miR-574-3p, were significantly expressed according to the selection criteria and were selected for further analysis.

### Validation study of miRNAs and their correlation with clinical parameters.

To confirm the diagnostic robustness of our discovered miRNA signature to discriminate between ST and SI cohorts, we then performed validation of these 10 miRNAs in a total population, 45 SI and 39 ST subjects. Our results showed that let-7c-5p, let-7d-5p, let-7f-5p, miR-376a-3p and miR-376c-3p were significantly increased in plasma of the SI versus ST cohort (Figure 2). We investigated the association between differentially expressed miRNAs and some clinical parameters in the SI group. Years of dyslipidemia (YDL) and non-HDL-c levels were selected as they show significant differences between SI and ST patients, and these differences could be related to the SI condition. No correlation was found between the selected clinical parameters with differentially expressed miRNAs (Table 2).

**Table 2. Correlation between clinical parameter (YDL and non-HDL-c) and individual microRNAs in IS subjects.**

| **microRNA** | **SI** | | | |
|---|---|---|---|---|
| | **YDL (years)** | | **Non-HDL-c (mg/dL)** | |
| | Pearson r | *p* | Pearson r | *p* |
| Let-7c-5p | -0.164 | 0.281 | 0.177 | 0.244 |
| Let-7d-5p | -0.079 | 0.603 | 0.226 | 0.131 |
| Let-7f-5p | -0.136 | 0.368 | 0.168 | 0.266 |
| miR-376a-3p | -0.173 | 0.250 | 0.103 | 0.498 |
| miR-376c-3p | -0.265 | 0.079 | 0.176 | 0.248 |

| | | | | |
|---|---|---|---|---|
| SI: statin intolerant; YDL: years of dyslipidemia. Coefficient significant at p < 0.05. | | | | |

### Circulating miRNA as a marker and early predictor of SI

We evaluated the ability of differentially expressed circulating miRNAs to discriminate between SI and ST patients using the AUC-ROC. For this purpose, we generated an ROC curve to assess the accuracy of let-7c-5p, let-7d-5p, let-7f-5p, miR-376a-3p and miR-376c-3p as plasma biomarkers for the detection of SI. Figure 3A shows that miR-376c-3p achieved the highest AUC with a value of 0.736 (95 % CI: 0.627-0.845; p < 0.001), indicating moderate performance in discriminating between SI and ST patients. Let-7c-5p, let-7d-5p, let-7f-5p and miR-376a-3p show AUC values of 0.652, 0.627, 0.688 and 0.682, respectively.

Subsequently, we considered the diagnostic potential of the 5-miRNA array to discriminate between SI and ST individuals. The AUC for the combination value of the selected miRNAs (let-7c-5p, let-7d-5p, let-7f-5p, miR-376a-3p and miR-376c-3p) was 0.936 (95 % CI: 0.887- 0.985; p < 0.001) (Figure 3B), demonstrating improved diagnostic performance. The sensitivity, specificity and accuracy of each miRNA and the 5-miRNA panel are shown in Table 3.

**Table 3. Evaluation of the potential of differentially expressed miRNAs and the panel of 5 miRNAs (let-7c-5p, let-7d-5p, let-7f-5p, miR-376a-3p and miR-376c-3p) for biomarkers.**

| **miRNA** | **AUC (95% CI)** | **Sensitivity %** | **Specificity %** | **Accuracy %** | ***p* value** |
|---|---|---|---|---|---|
| **Let-7c-5p** | 0.652 (0.535 a 0.770) | 61.70 | 55.56 | 59.04 | 0.017 |
| **Let-7d-5p** | 0.627 (0.507 a 0.747) | 52.63 | 58.70 | 55.95 | 0.046 |
| **Let-7f-5p** | 0.688 (0.573 a 0.803) | 60,53 | 64.44 | 62.65 | 0.003 |
| **miR-376a-3p** | 0.682 (0.563 a 0.800) | 68,89 | 64.10 | 66.67 | 0.004 |
| **miR-376c-3p** | 0.736 (0.627 a 0.845) | 70.45 | 64.10 | 67.47 | <0.001 |
| **5-miRNA panel** | 0.936 (0.887 a 0.985) | 81.25 | 84.85 | 82.72 | <0.001 |

| | | | | | |
|---|---|---|---|---|---|
| SI, statin intolerant; AUC, area under the curve; CI, confidence interval. | | | | | |

### Combination of differentially expressed miRNA, years of dyslipidemia (YDL) and non-HDL-c cholesterol concentration to diagnose patients with SI.

Years of dyslipidemia and plasma non-HDL cholesterol concentration were significantly higher in SI patients compared with ST (Figure 4A and B). We first evaluated the potential of these two clinical parameters to distinguish between the SI and ST groups. The ROC curves for years of dyslipidemia and non-HDL cholesterol concentration showed AUC values of 0.700 and 0.807, indicating moderate performance in discriminating SI from ST patients, whereas the combination of these two parameters reached an AUC value of 0.844 (Table 4). We further investigated whether the diagnostic performance for assessing patients with SI could be increased by using a multivariate model combining differentially expressed miRNA, years of dyslipidemia and/or non-HDL cholesterol. The diagnostic performance of the 5 miRNA panel was only slightly improved with the variable years of dyslipidemia since, although the AUC value was similar, this model achieved an accuracy of 84.81 % versus 82.72 % shown by the 5 miRNA panel (Figure 4D and E). In contrast, the diagnostic ability of the 5 miRNA panel together with non-HDL cholesterol concentration was lower than that of the 5 miRNA panel alone, as the ROC curve showed an AUC value of 0.855 (Figure 4D and Table 4). Interestingly, the highest diagnostic performance was achieved with the combination of the 3 miRNA panel composed of let-7f-5p, miR-376a-3p and miR-376c-3p, and years of dyslipidemia (YDL) plus non-HDL cholesterol concentration with an AUC value of 0.954 and an accuracy of 89.47% (Figure 4D and Table 4).

**Tabla 4. Evaluation of the potential of clinical parameters (YDL and non-HDL-c) and multivariate models as biomarkers.**

| SI, statin intolerant;AUC, area under the curve; CI, confidence interval; YDL, years of dyslipidemia Non-HDL-c, non-HDL-cholesterol; | | | | | |
|---|---|---|---|---|---|
| **Clinical parameter/model** | **AUC (95% CI)** | **Sensitivity %** | **Specificity %** | **Accuracy %** | ***p* value** |
| YDL (years) | 0.700 (0.587 a 0.814) | 57.89 | 60.00 | 58.54 | 0.017 |
| Non-HDL-c (mg/dL) | 0.807 (0.703 a 0.911) | 77.08 | 84.38 | 80.00 | <0.001 |
| YDL + non-HDL-c | 0.844 (0.751 a 0.937) | 79.55 | 85.29 | 82.05 | <0.001 |
| 5-miRNA panel + YDL | 0.940 (0.892 a 0.989) | 85.71 | 83.78 | 84.81 | <0.001 |
| 5-miRNA panel + non-HDL-c | 0.889 (0,814 a 0,964) | 85.00 | 81.08 | 83.12 | <0.001 |
| 3-miARN panel + YDL + non-HDL-c | 0.954 (0.911 a 0.998) | 89.74 | 89.19 | 89.47 | <0.001 |

### Gene ontology enrichment analysis of genes and KEGG pathways.

Next, we investigated the biological significance of the 5 differentially expressed miRNAs by Gene Ontology (GO) enrichment and Kyoto Encyclopedia of Genes and Genomes (KEGG) pathway analysis. We used miRNet online software (https://www.mirnet.ca) to predict the putative targets of 7c-5p, let-7d-5p, let-7f-5p, miR-376a-3p and miR-376c-3p (Figure 5A). The 7c-5p, let-7d-5p, let-7f-5p, miR-376a-3p, and miR-376c-3p were associated with 516, 394, 397,112, and 84 mRNAs in the miRNet database, respectively (Figure 5B). These 5 miRNAs shared only one mRNA target, IGF1R (Figure A and B). GO analysis using the computational tool WebGesalt (WEB-based GEne SeT Analysis Toolkit) showed significant enrichment of biological processes related to nerve growth factor response, cell cycle, gene silencing, and transforming growth factor beta (TGF-ß) response, among others (Figure 5C, left panel). KEGG pathway analysis showed several pathways, the most significant of which are the p53 signaling pathway, EGFR tyrosine kinase inhibitor resistance and glioma (Figure 5C, right panel). Finally, disease enrichment analysis identified that these 5 miRNAs might be involved in leukemia, mitochondrial myopathy, and (non-insulin-dependent) diabetes mellitus, among others (Figure 5C, bottom panel).

### References

Mach et al, 2020, "2019 ESC/EAS Guidelines for the management of dyslipidaemias: lipid modification to reduce cardiovascular risk," Eur Heart J, 2020 Jan 1; 41(1):111-188. doi: 10.1093/eurheartj/ehz455.
Townsend, N. et al., 2016, "Cardiovascular disease in Europe: epidemiological update 2016", European Heart Journal, Volume 37, Issue 42, 7 November 2016, Pages 3232-3245, doi.org/10.1093/eurheartj/ehw334.
Yusuf, S., et al., 2004, "Effect of potentially modifiable risk factors associated with myocardial infarction in 52 countries (the INTERHEART study): case-control study" Lancet, 2004. 364(9438): p. 937-952. doi: 10.1016/S0140-6736(04)17018-9.
Cholesterol Treatment Trialists' (CTT) Collaboration, et al., 2020, "Efficacy and safety of more intensive lowering of LDL cholesterol: a meta-analysis of data from 170,000 participants in 26 randomised trials," Lancet 376(9753):1670-81, doi: 10.1016/S0140-6736(10)61350-5.
Banach M. et al., 2016, "Statin non-adherence and residual cardiovascular risk: there is need for substantial improvement," I. J. Cardiol. 225:184-196. doi:10.1016/j.ijcard.2016.09.075.
Stulc T, Ceska R, Gotto Jr A., 2015, "Statin intolerance: The clinician's perspective," Curr Atheroscler Rep, 2015; 17: 69. doi: 10.1007/s11883-015-0552-3.
Guyton JR, Bays HE, Grundy SM, Jacobson TA, The National Lipid Association Statin Intolerance Panel, 2014, "An assessment by the Statin Intolerance Panel: 2014 update", J Clin Lipidol, 2014; 8: S72-S8121 doi: 10.1016/j.jacl.2014.03.002.
Wai MY, Ito MK, Cohen JD, Brinton EA, Jacobson TA, 2013, "Predictors of statin adherence, switching, and discontinuation in the USAGE survey: understanding the use of statins in America and gaps in patient education," J Clin Lipidol, 2013; 7: 472-484. doi: 10.1016/j.jacl.2013.03.001.
Rosenson, R. S., Baker, S. K., Jacobson, T. A., Kopecky, S. L. & Parker, B. A., 2014, "An assessment by the statin muscle safety task force: 2014 update", J. Clin. Lipidol. 8, S58-S71. 20.
Zhang H, et al., 2017, "Continued statin prescriptions after adverse reactions and patient outcomes: a cohort study," Ann Intern Med. 2017; 167:221-227. doi: 10.7326/M16-0838.
Takeshi Hirota, Yuito Fujita & Ichiro leiri. 2020. "An updated review of pharmacokinetic drug interactions and pharmacogenetics of statins. "Expert Opinion on Drug Metabolism & Toxicology, 16:9, 809-822, doi: 10.1080/17425255.2020.1801634.
Ubilla Carmen Gloria, Prado Yalena, Angulo Jeremy, Obreque Ignacio, Paez Isis, Saavedra Nicolás, Saavedra Kathleen, Zambrano Tomás, Salazar Luis A. 2021. "MicroRNA-33b is a Potential Non-Invasive Biomarker for Response to Atorvastatin Treatment in Chilean Subjects With Hypercholesterolemia: A Pilot Study". Frontiers in Pharmacology 12, 2021. doi:10.3389/fphar.2021.674252.
Arrigoni, Elena, Marzia Del Re, Leonardo Fidilio, Stefano Fogli, Romano Danesi, and Antonello Di Paolo. 2017. "Pharmacogenetic Foundations of Therapeutic Efficacy and Adverse Events of Statins" International Journal of Molecular Sciences 18, no. 1: 104. https://doi.org/10.3390/ijms18010104.
Shun Saito, Takeo Nakanishi, Yuma Shirasaki, Miki Nakajima, Ikumi Tamai. 2017 "Association of miR-145 With Statin-Induced Skeletal Muscle Toxicity in Human Rhabdomyosarcoma RD Cells" Research Article Pharmacokinetics, Pharmacodynamics and Drug Transport and Metabolism, volume 106, issue 9, p2873-2880, september 01, 2017 https://doi.org/10.1016/j.xphs.2017.04.005
Tomás Zambrano, Rosario D.C. Hirata, Mario H. Hirata, Alvaro Cerda, Luis A. Salazar. 2018 "Statins differentially modulate microRNAs expression in peripheral cells of hyperlipidemic subjects: A pilot study. "European Journal of Pharmaceutical Sciences, Volume 117, 2018, Pages 55-61, https://doi.org/10.1016/j.ejps.2018.02.007.
David P, B., 2004, "MicroRNAs: Genomics, Biogenesis, Mechanism, and Function", Cell. Volume 116, Issue 2, 23 January 2004, Pages 281-297. https://doi.org/10.1016/S0092-8674(04)00045-5.
He et al., 2004, "MicroRNAs: small RNAs with a big role in gene regulation" Nat. Rev. Genet. 2004 Jul; 5 (7): 522-31. doi: 10.1038/nrg1379.
Stepie'n E., 2018, "The circulating non-coding RNA landscape for biomarker research: lessons and prospects from cardiovascular diseases", Acta Farmacologica Sinica, 39, pages1085-1099. doi.org/10.1038/aps.2018.35.
Lu D. et al., 2019, "RNA-based diagnostic and therapeutic strategies for cardiovascular disease," Nat. Rev. Cardiol. 16:661-674.
Belmonte et al, 2020, "Emerging role of microRNAs in dilated cardiomyopathy: evidence regarding etiology," Transl Res. 2020 Jan; 215:86-101. doi: 10.1016/j.trsl.2019.08.007. Epub 2019 Aug 23. PMID: 31505160.
Huaiyu M et al., 2019, "PANTHER version 14: more genomes, a new PANTHER GO-slim and improvements in enrichment analysis tools", Nucleic Acids Research, Volume 47, Issue D1, Pages D419-D426. https://doi.org/10.1093/nar/gky1038.
Szklarczyk, D. et al., 2019, "STRING v11: protein-protein association networks with increased coverage, supporting functional discovery in genome-wide experimental datasets", Nucleic Acids Research, Volume 47, Issue D1, Pages D607-D613. https://doi.org/10.1093/nar/gky1131.
Andersen, C.L.; Jensen, J.L.; Orntoft, T.F. 2004 "Normalization of real-time quantitative reverse transcription-PCR data: A model-based variance estimation approach to identify genes suited for normalization, applied to bladder and colon cancer data sets". Cancer Res. 2004, 64, 5245-5250.

## Claims

1. Use *in vitro* of let-7c-5p, let-7d-5p, let-7f-5p, miR-376a-3p and/or miR-376c-3p expression levels as biomarkers to diagnose statin intolerance in patients with cardiovascular risk, where overexpression of these biomarkers with respect to the mean values of a normal individual allows assigning an individual to the group of patients who will suffer adverse effects derived from statin treatment.

2. The use according to claim 1 wherein the biomarkers let-7c-5p, let-7d-5p, let-7f-5p, miR-376a-3p and miR-376c-3p are used simultaneously.

3. The use according to claim 1 wherein the biomarkers let-7f-5p, miR-376a-3p and miR-376c-3p are used simultaneously.

4. A biomarker expression profile suitable for diagnosing statin intolerance comprising expression levels of let-7c-5p, let-7d-5p, let-7f-5p, miR-376a-3p and/or miR-376c-3p, where overexpressed levels relative to average values for a normal individual is indicative of statin intolerance.

5. The biomarker expression profile according to claim 4 further comprising clinical variables to be selected from years of dyslipidemia, the presence of atherosclerotic cardiovascular disease, arterial hypertension, and/or non-HDL cholesterol levels.

6. The biomarker expression profile according to claim 5, wherein the biomarkers let-7c-5p, let-7d-5p, let-7f-5p, let-7f-5p, miR-376a-3p and miR-376c-3p are used in combination and the selected clinical variables are years of dyslipidemia and/or non-HDL cholesterol levels, wherein values above those of a normal individual in these clinical variables is indicative of statin intolerance.

7. The biomarker expression profile of claim 5, wherein the selected biomarkers are let-7f-5p, miR-376a-3p and miR-376c-3p in combination and the selected clinical variables are years of dyslipidemia and non-HDL cholesterol levels, wherein values above that of a normal individual in these clinical variables is indicative of statin intolerance.

8. Method *in vitro* of obtaining data useful for predicting or prognosticating statin intolerance in individuals with cardiovascular risk comprising:
a) obtaining an isolated biological sample from the individual,
b) quantifying the expression levels of let-7c-5p, let-7d-5p, let-7f-5p, miR-376a-3p and miR-376c-3p, or any of their combinations.

9. The method *in vitro* of obtaining useful data according to claim 8 further comprising:
c) comparing the quantities obtained in step (b) with a reference quantity.

10. The method *in vitro* of obtaining useful data according to claim 7 wherein step b) quantifies the amount of expression product of let-7c-5p, let-7d-5p, let-7f-5p, miR-376a-3p and miR-376c-3p simultaneously.

11. The method *in vitro* of obtaining useful data according to claim 7 wherein step b) quantifies the amount of let-7f-5p, miR-376a-3p and miR-376c-3p expression product simultaneously.

12. The method *in vitro* of obtaining useful data according to claims 7 -11 wherein steps (b) and/or (c) can be fully or partially automated.

13. The method *in vitro* for predicting or forecasting statin intolerance in individuals with cardiovascular risk comprising steps (a) - (c) of claim 8 and further comprising:
d) assigning the individual who presents in an analysis, before administering statin treatment, the expression levels of let-7c-5p, let-7d-5p, let-7f-5p, miR-376a-3p and/or miR-376c-3p overexpressed with respect to the mean values of a normal individual, to the group of patients who will suffer adverse effects derived from statin treatment.

14. The method according to claim 13 wherein the expression levels of the biomarkers let-7c-5p, let-7d-5p, let-7f-5p, miR-376a-3p and miR-376c-3p are simultaneously overexpressed.

15. The method according to claim 13 wherein the levels of let-7f-5p, miR-376a-3p and miR-376c-3 are simultaneously overexpressed.

16. The method according to claims 8 to 15, wherein the biological sample is selected from blood, plasma and serum.

17. The method according to any one of claims 8 to 16, wherein the biological sample is plasma.

18. The method according to any one of claims 8 to 17, wherein said result is obtained by:
(i) a method of generating miRNA profiles, such as a microarray, and/or
(ii) a method comprising PCR (polymerase chain reaction), such as real-time PCR;
(iii) Northern transfer and/or
(iv) immunoassay.

19. A kit comprising at least one or more oligonucleotides capable of hybridizing to let-7c-5p, let-7d-5p, let-7f-5p, miR-376a- 3p and/or miR-376c-3p, or any combination thereof, and elements necessary to quantify in a biological sample obtained from the subject the expression levels of said biomarkers.

20. The kit according to claim 19 comprising at least one or more oligonucleotides capable of simultaneously hybridizing let-7c-5p, let-7d-5p, let-7f-5p, miR-376a- 3p and miR-376c-3p.

21. The kit according to claim 19 comprising at least one or more oligonucleotides capable of simultaneously hybridizing let-7f-5p, miR-376a-3p and miR-376c-3p.

22. The kit according to claims 19 to 21 further comprising means for comparing the expression level of the biomarkers with a reference sample.

23. The kit according to claim 22 further comprising instructions for a medical professional to administer the statin treatment only to those subjects who can be classified in the group of non-statin intolerant.

24. Use of the kit according to claims 19 to 23, for predicting or forecasting intolerance to statin therapy in an individual with cardiovascular risk.

25. A computer program adapted for any processing means to implement the method according to any one of claims 8-18.

26. A computer-readable storage medium comprising program instructions capable of causing a computer to carry out the steps of the method according to any one of claims 8-18.

27. A transmittable signal comprising program instructions capable of causing a computer to carry out the steps of the method according to any one of claims 8-18.
